# EUROPÄISCHE PATENTSCHRIFT

(11) **EP 1 010 689 B1**
(45) Veröffentlichungstag und Bekanntmachung des Hinweises auf die Patenterteilung: **15.01.2003**
(21) Anmeldenummer: 99122437.9
(22) Anmeldetag: 11.11.1999
(51) Int. Cl.: C07C 215/64, A61K 31/135

(54) **Substituierte Cycloheptene, deren Herstellung und Verwendung**
Substituted cycloheptenes, their preparation and use
Cycloheptènes substitués, production et utilisation

(30) Priorität: 14.12.1998 DE 19857475
(43) Veröffentlichungstag der Anmeldung: 21.06.2000
(73) Patentinhaber: Grünenthal GmbH, 52078 Aachen (DE)
(72) Erfinder: Zimmer, Oswald, Dr., 52146 Würselen (DE); Strassburger, Wolfang Werner Alfred, Prof. Dr., 52146 Würselen (DE); Englberger, Werner Günter, Dr., 52223 Stolberg (DE); Kögel, Babette-Yvonne, Dr., 52379 Langerwehe-Hamich (DE)

(56) Entgegenhaltungen:
- DE-A- 1 518 663
- US-A- 3 830 934

## Beschreibung

Die Erfindung betrifft substituierte Cycloheptene der allgemeinen Formel (I) 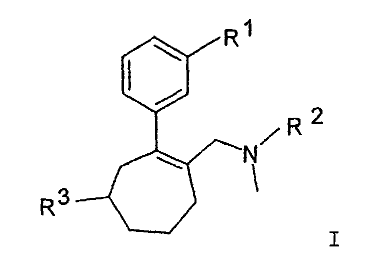 worin
- R¹: OH, O-(C₁₋₆)-Alkyl, O-(C₃₋₇)Cycloalkyl, O-Aryl, C₁₋₆-Alkyl-COO-, Aryl-COO-,
- R²: C₁₋₆-Alkyl, (CH₂)₍₁₋₂₎-Aryl, C₂₋₆-Alkenylen-Aryl und
- R³: (CH₂) ₍₀₋₁₎-C₅₋₇-Cycloalkyl, (CH₂) ₍₀₋₂₎-Aryl, Heterocyclyl, C₁₋₆-Alkyl-Heterocyclyl,
bedeuten oder oder als Racemat oder in Form reiner Enantiomere vorliegen, jeweils als Base oder als Salz mit einer pharmazeutisch verwendbaren Säure, ein Verfahren zu deren Herstellung sowie ihre Verwendung als Arzneimittel.

Klassische Opioide wie das Morphin sind bei der Therapie starker bis stärkster Schmerzen gut wirksam. Ihr Einsatz wird jedoch durch die bekannten Nebenwirkungen, z. B. Atemdepression, Erbrechen, Sedierung und Obstipation und die Toleranzentwicklung eingeschränkt. Außerdem sind sie bei neuropathischen oder inzidentiellen Schmerzen, unter denen insbesondere Tumorpatienten leiden, weniger wirksam.

Opioide entfalten ihre analgetische Wirkung durch Bindung an membranständige Rezeptoren, die zur Familie der sogenannten G-Protein-gekoppelten Rezeptoren gehören. Die biochemische und pharmakologische Charakterisierung von Subtypen dieser Rezeptoren hat nun die Hoffnung geweckt, daß subtypenspezifische Opioide über ein anderes Wirkungs-Wirkungs-/Nebenwirkungsprofil als z. B. Morphin verfügen. Während Morphin selektiv an die sogenannten µ-Rezeptoren bindet, wurden die endogenen Enkephaline als δ-selektive Peptide charakterisiert. Weitere pharmakologische Untersuchungen haben inzwischen die Existenz mehrerer Subtypen dieser Opioidrezeptoren (µ₁, µ₂, κ₁, κ₂, κ₃, δ₁ und δ₂) wahrscheinlich gemacht.

Kenntnisse über die physiologische Bedeutung von δ-rezeptorselektiven Substanzen sind wesentlich durch die Entdeckung des nicht-peptidischen Antagonisten Naltrindol erweitert worden. So steht inzwischen fest, daß δ-Agonisten über ein eigenständiges antinociceptives Potential verfügen. Neben einer Vielzahl von tierexperimentellen Studien gibt es dazu auch eine Untersuchung mit dem peptidischen Agonisten D-Alanin²-D-Leucin⁵-Enkephalin (DADL) an Krebspatienten, bei denen Morphin keine analgetische Wirkung mehr hatte. Bei intrathekaler Gabe zeigte DADL einen lang anhaltenden analgetischen Effekt.

Deutlich unterscheiden sich δ- von µ-Agonisten in ihrer Wechselwirkung mit dem "endogenen Opioidantagonisten" Cholecystokinin (CCK). Neben diesem unterschiedlichen Wirkprofil könnte sich auch das Nebenwirkungsprofil der δ-Agonisten von µ-Agonisten unterscheiden, z. B. durch Verminderung der Atemdepression oder der Obstipation. Potentiell therapeutisch einsetzbar sind diese Verbindungen als Analgetika und, ganz allgemein, für alle pathologischen Zustände, die gewöhnlich mit δ-Opiatrezeptoragonisten behandelt werden können.

Die der Erfindung zugrundeliegende Aufgabe bestand deshalb darin, analgetisch wirksame Substanzen zu finden, deren biologische Wirksamkeit teilweise oder überwiegend über δ-Opiatrezeptoragonisten vermittelt wird.

Es wurde nun gefunden, daß diese Anforderungen von den substituierten Cycloheptenverbindungen der allgemeinen Formel (I) erfüllt werden.

Gegenstand der vorliegenden Erfindung sind neue substituierte Cycloheptene der allgemeinen Formel I, 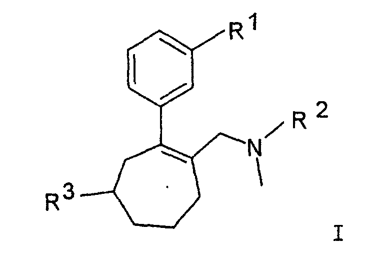 worin
- R¹: OH, O-(C₁₋₆)-Alkyl, O-(C₃₋₇)Cycloalkyl, O-Aryl, C₁₋₆-Alkyl-COO-, Aryl-COO-,
- R²: C₁₋₆-Alkyl, (CH₂) (CH₂)₍₁₋₂₎-Aryl, C₂₋₆-Alkenylen-Aryl und
- R³: (CH₂)₍₀₋₁₎-C₅₋₇-Cycloalkyl, (CH₂)₍₀₋₂₎-Aryl, Heterocyclyl, C₁₋₆-Alkyl-Heterocyclyl
bedeuten oder in Form ihrer Enantiomeren, Diastereomeren, Racemate, Basen oder als Salze von physiologisch verträglichen Säuren vorliegen.

Bevorzugt sind Verbindungen der allgemeinen Formel I, in denen R¹ OH, O-(C₁₋₆)-Alkyl, O-(C₃₋₇)Cycloalkyl, O-Aryl, C₁₋₆-Alkyl-COO- oder Aryl-COO-, und R² bis R³ die Bedeutung gemäß der Definition der allgemeinen Formel I haben oder
R¹ OH, O-(C₁₋₆)-Alkyl oder O-(C₃₋₇)Cycloalkyl, R² C₁₋₆-Alkyl oder (CH₂)₍₁₋₂₎-Aryl und R³ die Bedeutung gemäß der Definition der allgemeinen Formel I haben oder
R¹ OH, R² C₁₋₆-Alkyl oder (CH₂)₍₁₋₂₎-Aryl und R³ die Bedeutung gemäß der Definition der allgemeinen Formel I haben oder
R¹ OH, R² C₁₋₆-Alkyl und R³ die Bedeutung gemäß der Definition der allgemeinen Formel I haben.

Zu weiteren bevorzugten Verbindungen zählen:
3- [6-(4-Chlor-phenyl)-2-dimethylaminomethyl-cyclohept-1-enyl]-phenol, Hydrochlorid,
3-(2-Dimethylaminomethyl-6-phenyl-cyclohept-1-enyl)-phenol, Hydrochlorid,
3-(2-Dimethylaminomethyl-6-naphth-1-yl-cyclohept-1-enyl)-phenol, Hydrochlorid,
3-(2-Dimethylaminomethyl-6-naphth-2-yl-cyclohept-1-enyl)-phenol, Hydrochlorid,
3-[2-Dimethylaminomethyl-6-(4-hydroxy-phenyl)-cyclohept-1-enyl]-phenol, Hydrochlorid,
3-(2-Dimethylaminomethyl-6-m-tolyl-cyclohept-1-enyl)-phenol, Hydrochlorid,
3-[6-(3-tert-Butyl-phenyl)-2-dimethylaminomethyl-cyclohept-1-enyl]-phenol, Hydrochlorid,
6-[4-Dimethylaminomethyl-3-(3-hydroxy-phenyl)-cyclohept-3-enyl]-naphth-2-ol, Hydrochlorid,
3-[2-Dimethylaminomethyl-6-(3-fluor-4-hydroxy-phenyl)-cyclohept-1-enyl]-phenol, Hydrochlorid,
3-[2-Dimethylaminomethyl-6-(2-hydroxy-phenyl)-cyclohept-1-enyl]-phenol, Hydrochlorid,
3-(6-Cyclohexyl-2-dimethylaminomethyl-cyclohept-1-enyl)-phenol, Hydrochlorid,
3-(6-Cyclohexylmethyl-2-dimethylaminomethyl-cyclohept-1-enyl)-phenol, Hydrochlorid,
3-(6-Benzyl-2-dimethylaminomethyl-cyclohept-1-enyl)-phenol, Hydrochlorid,
3-[2-Dimethylaminomethyl-6-(3-hydroxy-benzyl)-cyclohept-1-enyl)-phenol, Hydrochlorid,
3-(2-Dimethylaminomethyl-6-phenethyl-cyclohept-1-enyl)-phenol, Hydrochlorid,
3-[2-Dimethylaminomethyl-6-(3,5-dimethyl-4-hydroxyphenyl)-cyclohept-1-enyl]-phenol, Hydrochlorid,
3-[2-Dimethylaminomethyl-6-(3-hydroxy-phenyl)-cyclohept-1-enyl]-phenol, Hydrochlorid,
3-{2-[(Methyl-phenethyl-amino)-methyl]-6-phenyl-cyclohept-1-enyl}-phenol, Hydrochlorid und
[2-(3-Methoxy-phenyl)-4-naphth-1-yl-cyclohept-1-enylmethyl]-dimethylamin, Hydrochlorid.

Der Ausdruck "C₁₋₆-Alkyl" bedeutet in der vorliegenden Erfindung geradkettige oder verzweigte Kohlenwasserstoffe mit 1-6 Kohlenstoffatomen. Beispielhaft seien Methyl, Ethyl, Propyl, Isopropyl, n-Butyl, sek-Butyl, tert-Butyl, n-Pentyl, Neopentyl und n-Hexyl genannt.

Im Rahmen der vorliegenden Erfindung bedeutet der Ausdruck "C₂₋₆-Alkenylen" geradkettige oder verzweigte Kohlenwasserstoffreste mit 2 bis 6 Kohlenstoffatomen, mit einer oder mehreren Doppelbindungen enthalten können. Beispielhaft seien 2-Propenyl, 2-Butenyl, 1-Methyl-2-propenyl, 2-Methyl-2-propenyl,, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Diemthyl-2-propenyl, 2-Hexenyl, 3-Hexenyl, 4-Hexenyl, 5-Hexenyl, 1-Methyl-2-pentenyl oder 1,3-Dimethyl-3-butenyl erwähnt.

Der Ausdruck "Aryl" bedeutet im Rahmen der vorliegenden Erfindung unsubstituierte oder ein- oder mehrfach mit OH, F, Cl, CF₃, C₁₋₆-Alkyl, C₁₋₆-Alkoxy, C₁₋₇-Cycloalkoxy, C₃₋₇-Cycloalkyl, C₂₋₆-Alkenylen, Heterocyclyl oder Phenyl substituierte Phenyle. Die Heterocyclyl - oder Phenylreste können gegebenenfalls ankondensiert sein. Der Ausdruck kann gegebenenfalls auch Naphthyl bedeuten.

Unter dem Ausdruck "Heterocyclyl" sind im Rahmen der vorliegenden Erfindung 5- oder 6-gliedrige gesättigte oder ungesättigte, gegebenenfalls mit einem ankondensierten Arylsystem versehene, heterocyclische Verbindungen zu verstehen, die 1 oder 2 Heteroatome aus der Gruppe von Stickstoff, Sauerstoff und/oder Schwefel enthalten.

Beispielhaft seien für die gesättigten Heterocyclyle 1,4-Dioxan, Tetrahydrofuran und 1,4-Thioxan aufgeführt.

Aus der Gruppe der ungesättigten Heterocyclyle seien beispielhaft Furan, Thiophen, Pyridin, Pyrimidin, Thiazol, Oxazol, Isoxazol, Pyridazin, Pyrazin, Chinolin, Isochinolin, Phthalazin und Chinazolin aufgeführt.

Der Ausdruck "C₁₋₆-Alkyl-Heterocyclyl" bedeutet im Rahmen der vorliegenden Erfindung, daß die "Heterocyclyle" wie oben definiert über eine C₁₋₆-Alkyl-Gruppe gebunden sind. Der Ausdruck "C₂₋₆-Alkenylen-Aryl" bedeutet im Rahmen der vorliegenden Erfindung, daß die Aryle wie oben definiert über eine C₂₋₆-Alkenylen-Gruppe gebunden sind.

Unter dem Ausdruck "Silanylverbindung" sind im Rahmen der vorliegenden Erfindung Trialkyl- oder Triarylsilyle, Dialkylarylsilyle oder Diarylalkylsilyle zu verstehen, die als Schutzgruppe für die Hydroxy-Funktion verwendet werden. Beispielhaft seien Triethylsilyl, Tripropylsilyl, Dimethyl-phenylsilyl, Di-tert-butylphenylsilyl, Triisopropylsilyl, Dimethylisopropylsilyl, Diethylisopropylsilyl, Dimethylthexylsilyl, tert-Butyldimethylsilyl, tert-Butyldiphenylsi-lyl, Tribenzylsilyl, Tri-p-xylylsilyl, Triphenylsilyl, Diphenylmethylsilyl oder Propyl-diphenylsilyl erwähnt.

Erfindungsgegenstand ist auch ein Verfahren zur Herstellung der Verbindungen der allgemeinen Formel I, das gekennzeichnet ist durch die Umsetzung eines tertiären Alkohols der allgemeinen Formel II, 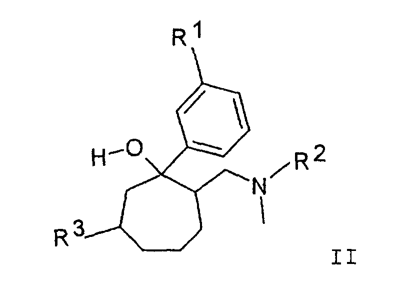 in der R¹ bis R³ dieselbe Bedeutung wie in Formel I haben, mit halbkonzentrierten oder konzentrierten organischen oder anorganischen Säuren wie z. B. Salzsäure, Bromwasserstoffsäure, Ameisensäure oder Lösungen von Bromwasserstoff in Essigsäure bei Temperaturen von 20°C bis 110°C, wobei die tertiären Alkohole der Formel II dadurch erhalten werden, daß Aminoketone der allgemeinen Formel III 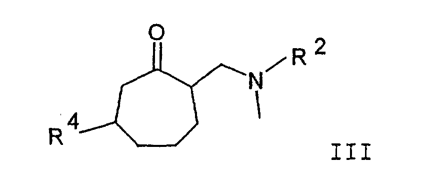 wobei R² wie oben definiert ist und R⁴ dieselbe Bedeutung wie R³ hat, mit der Ausnahme, daß eine gegebenenfalls vorhandene Hydroxyfunktion in geschützter Form, die etwa als Benzyloxy- oder Silanyloxygruppe vorliegt, mit einer metallorganischen Verbindung der Formel IV, 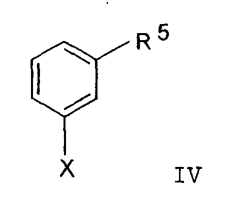 in der X für MgCl, MgBr, MgI oder Li steht und R⁵ die Bedeutung gemäß R¹ besitzt, mit der Ausnahme, daß wie in R⁴ eine ggf. vorhandene Hydroxyfunktion in geschützter Form, die etwa als Benzyloxy- oder Silanyloxygruppe vorliegt, zu einer Verbindung der allgemeinen Formel IIa umgesetzt werden, 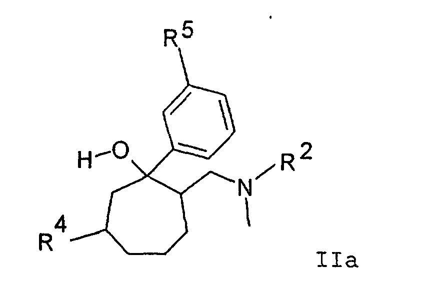 die dann in eine Verbindung der allgemeinen Formel II übergeführt wird.

Verbindungen der allgemeinen Formel III erhält man aus Cycloheptanonen der allgemeinen Formel V, 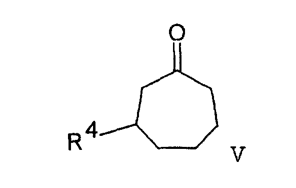 in der R⁴ dieselbe Bedeutung wie oben angegeben hat, durch Umsetzung mit Aminen der allgemeinen Formel HN(CH₃)R² (ggf. in Form ihrer Salze) und Paraformaldehyd bzw. wäßriger Formaldehydlösung in Lösemitteln wie Wasser, Alkoholen oder Essigsäure bei Temperaturen zwischen 20°C und der Siedetemperatur des Lösemittels. Vorzugsweise erfolgt die Herstellung der Verbindung der allgemeinen Formel III jedoch durch Umsetzung von V mit Methylenimmoniumhalogeniden der allgemeinen Formel H₂C=N(CH₃)R²X, wobei R² wie oben definiert ist und X für ein Chlor- oder Jodatom steht, in Lösemitteln wie Acetonitril oder Tetrahydrofuran bei Temperaturen von 20°C bis 50°C.

Die Umsetzung der Verbindungen III und IV wird in einem aliphatischen Ether, beispielsweise Diethylether und/oder Tetrahydrofuran, bei Temperaturen von -70°C bis +60°C durchgeführt. Verbindungen der Formel IV, in denen X für ein Lithiumatom steht, werden dabei aus Verbindungen der Formel IV, in denen X Br oder I bedeutet, durch Halogen-Lithiumaustausch mittels z. B. einer n-Butyllithium/n-Hexan-Lösung gewonnen.

Für die Umsetzung einer Verbindung der Formel IIa zu einer der Formel II stehen in Abhängigkeit von R⁵ bzw. der Schutzgruppe in R⁴ mehrere Methoden zur Verfügung.

Steht R⁵ für eine Benzyloxygruppe und/oder ist in R⁴ eine solche enthalten, so geschieht dies zweckmäßig durch eine reduktive Debenzylierung mit katalytisch aktiviertem Wasserstoff, wobei Platin oder Palladium, absorbiert auf einem Trägermaterial wie Aktivkohle, als Katalysator dienen. Die Reaktion wird in einem Lösungsmittel wie Essigsäure oder einem C₁₋₄-Alkylalkohol bei Drücken von 1 bis 100 bar und Temperaturen von +20°C bis +100°C durchgeführt, wobei die Verbindung IIa vorzugsweise in Form eines ihrer Salze eingesetzt wird.

Stellt R⁵ eine Silanyloxygruppe dar und/oder ist in R⁴ eine solche enthalten, so erfolgt die Abspaltung der Schutzgruppe dadurch, daß man die entsprechende Verbindung der Formel IIa bei +20°C in einem inerten Lösemittel wie Tetrahydrofuran, Dioxan oder Diethylether mit Tetra-n-butylammoniumfluorid umsetzt oder mit einer methanolischen Lösung von Chlorwasserstoff behandelt.

Steht R⁵ für und/oder enthält R⁴ in der Verbindung der Formel IIa einen Methoxyrest, läßt sich durch Umsetzung mit Diisobutylaluminiumhydrid in einem aromatischen Kohlenwasserstoff wie Toluol bei einer Temperatur zwischen 60°C und 130°C die Verbindung der Formel II herstellen, in der R¹ für eine Hydroxygruppe steht und/oder R³ eine solche enthält. Man kann in diesem Fall auch direkt die analoge Verbindung der Formel I erhalten, indem man IIa entweder mit einer Lösung von Bromwasserstoff in Eisessig oder konzentrierter Bromwasserstoffsäure erhitzt. Dies ist auch durch Umsetzung von IIa mit Methansulfonsäure/Methionin /Methionin bei Temperaturen zwischen 20°C und 50°C möglich.

Auch in Verbindungen der Formel I, in denen R¹ eine Methoxygruppe darstellt und/oder in R³ eine solche enthalten ist, läßt sich diese wie oben beschrieben durch Umsetzung mit Diisobutylaluminiumhydrid in die Hydroxyfunktion umwandeln.

Verbindungen der allgemeinen Formel I, in denen R¹ eine Hydroxyfunktion darstellt, können in an sich bekannter Weise in eine Esterfunktion überführt werden.

Die Verbindungen der Formel I lassen sich mit physiologisch verträglichen Säuren wie Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure und/oder Asparaginsäure in an sich bekannter Weise in ihre Salze überführen. Vorzugsweise wird die Salzbildung in einem Lösungsmittel wie Diethylether, Diisopropylether, Essigsäurealkylester, Aceton und/oder 2-Butanon durchgeführt. Zur Herstellung der Hydrochloride ist Trimethylchlorsilan in wasserhaltiger Lösung besonders geeignet.

### δ-Opiatrezeptor-Bindungsuntersuchungen

Die Untersuchungen zur Bestimmung der Affinität der erfindungsgemäßen Verbindungen der Formel I zum δ-Opiatrezeptor wurde an Hirnmembranhomogenaten (Homogenat von Rattenhirn ohne Cerebellum, Pons und Medulla oblongata von männlichen Wistar-Ratten) durchgeführt. Hierzu wurde das jeweils frisch präparierte Rattenhirn unter Eiskühlung in 50 mmol/l Tris-HCl(Tris-(hydroxymethyl)-aminomethan-hydrochlorid) (pH 7,4) homogenisiert und für 10 Minuten bei 5.000 g und 4°C zentrifugiert. Nach Dekantieren und Verwerfen des Überstandes, erneutem Aufnehmen und Homogenisieren des Membransedimenbes in 50 mmol/l Tris-HCl (pH 7,4) wurde das Homogenat anschließend für 20 Minuten bei 20.000 g und 4° C zentrifugiert. Dieser Waschschritt wurde nochmals wiederholt. Danach wurde der Überstand dekantiert und das Membransediment in kaltem 50 mmol/l Tris-HCl , 20 % Glycerol (w/v), 0,01 % Bacitracin (w/v) (pH 7,4) homogenisiert und in Aliquoten bis zur Testung eingefroren. Für die Rezeptorbindungstestung wurden die Aliquote aufgetaut und 1:10 mit dem Bindungstest-Puffer verdünnt. Im Bindungstest wurde als Puffer ein 50 mmol/l Tris-HCL, 5 mmol/l MgCl₂ (pH 7, 4) supplementiert mit 0,1 % (w/v) bovinem Serumalbumin, sowie als radioaktiver Ligand 1 nmol/l (³H)-2-D-Ala-Deltorphin II eingesetzt. Der Anteil an unspezifischer Bindung wurde in Anwesenheit von 10 mmol/l Naloxon bestimmt. In weiteren Ansätzen wurden die erfindungsgemäßen Verbindungen in Konzentrationsreihen zugegeben und die Verdrängung des radioaktiven Liganden aus seiner spezifischen Bindung ermittelt. Die jeweiligen Dreifachansätze wurden über 90 Minuten bei 37° C inkubiert und anschließend zur Bestimmung des an das Membranhomogenat gebundenen radioaktiven Liganden mittels Filtration durch Glasfaserfilter (GF/B) geerntet. Die Radioaktivität der Glasfaserfilterscheiben wurde nach Zugabe von Szintillator im β-Counter gemessen. Die Affinität der erfindungsgemäßen Verbindungen zum δ-Opiatrezeptor wurde als IC₅₀ nach dem Massenwirkungsgesetz mittels nichtlinearer Regression berechnet. Die Kᵢ-Werte in Tabelle 1 sind als Mittelwert ± Standardabweichungen von 3 voneinander unabhängigen Versuchen angegeben.

**Tabelle 1**

| Beisp.-Nr. | δ-Optiatrezeptorbindung Kᵢ [nmol/l] |
|---|---|
| 1 | 1,4 ± 0,8 |
| 2a | 30,3 ± 4,7 |
| 2b | 3,8 ± 0,2 |
| 2c | 24,7 ± 2,4 |
| 2d | 31,5 ± 5,9 |
| 2e | 15,2 ± 4,3 |
| 2f | 3,2 ± 0,8 |
| 2g | 17,5 ± 5,2 |
| 2h | 19,4 ± 4,7 |
| 2i | 14,6 ± 2,2 |
| 2j | 24,7 ± 3,1 |
| 2k | 10,3 ± 2,2 |
| 2l | 28,6 ± 5,8 |
| 2m | 10,2 ± 1,0 |
| 2n | 7,4 ± 2,2 |
| 2O | 30,6 ± 10,5 |
| 2p | 2,5 ± 0,7 |
| 3 | 8,3 ± 3,3 |

### Prüfung auf antinociceptive Aktivität im Writhing-Test an der Maus

Die antinociceptive Wirksamkeit wurde im Phenylchinoninduzierten Writhing an der Maus, modifiziert nach I.C. Hendershot, J. Forsaith, J. Pharmacol. Exp. Ther. 125, 237 - 240 (1959) untersucht. Dazu wurden männliche NMRI-Mäuse mit einem Gewicht von 25 - 30 g eingesetzt. Gruppen von 10 Tieren pro Substanzdosis wurden 10 Minuten nach intravenöser Gabe einer erfindungsgemäßen Verbindung 0,3 ml/Maus einer 0,02 %igen wäßrigen Lösung von Phenylchinon (Phenylbenzochinon, Fa. Sigma, Deisenhofen; Herstellung der Lösung unter Zusatz von 5 % Ethanol und Aufbewahrung im Wasserbad bei 45°C) intraperitoneal appliziert. Die Tiere wurden einzeln in Beobachtungskäfige gesetzt. Mittels eines Drucktastenzählers wurde die Anzahl der Schmerz-induzierten Streckbewegungen (sogenannte Writhingreaktionen = Durchdrücken des Körpers mit Abstrecken der Hinterextremitäten) 5 - 20 Minuten nach der Phenylchinon-Gabe ausgezählt. Aus der dosisabhänigigen Abnahme der Writhingreaktionen im Vergleich zu parallel untersuchten Tiergruppen, denen keine erfindungsgemäße Verbindung appliziert wurde, wurden mittels Regressionsanalyse (Auswerteprogramm Martens EDV Service, Eckental) die ED₅₀-Werte der Writhingreaktion berechnet.

**Tabelle 2**

| Beisp.-Nr. | Writhing-Test, Maus ED₅₀ i.v. [mg/kg] (95 % Vertrauensbereich) |
|---|---|
| 1 | 3,31 (2,70 - 3,88) |
| 2a | 7,40 (5,53 - 9,21) |
| 2b | 4,48 (3,36 - 6,27) |
| 2c | 5,29 (4,11 - 7,17) |
| 2d | 7,56 (5,49 - 10,70) |
| 2e | 2,25 (1,56 - 3,00) |
| 2h | 6,22 (4,68 - 8,28) |
| 2i | 0,89 (0,62 - 1,29) |
| 3 | 3,40 (2,40 - 4,92) |

### Beispiele

Die folgenden Beispiele dienen zur näheren Erläuterung der vorliegenden Erfindung ohne sie jedoch einzuschränken.

Als stationäre Phase für die Säulenchromatographie wurde Kieselgel 60 (0.040 - 0.063 mm) der Firma E. Merck, Darmstadt, eingesetzt.

Die dünnschichtchromatographischen Untersuchungen wurden mit HPTLC-Fertigplatten, Kieselgel 60 F 254, der Firma E. Merck, Darmstadt, durchgeführt.

Die Mischungsverhältnisse der Laufmittel für alle chromatographischen Untersuchungen sind stets in Volumen/Volumen angegeben.

Der Ausdruck TRIS-HCl bedeutet Tris-(hydroxyinethyl)-aminomethan-hydrochlorid.
(w/v) Gewicht/Volumen

### Beispiel 1

### 3-[2-Dimethylaminomethyl-6-(3-hydroxy-phenyl)-cyclohept-1-enyl]-phenol, Hydrochlorid

### 1. Stufe

### (3-Methoxy-phenyl)-cycloheptanon

Zu einer frisch bereiteten Grignard-Lösung aus 5,83 g Magnesiumspänen und 28,7 ml 1-Brom-3-methoxy-benzol in 675 ml wasserfreiem Diethylether wurden bei 20°C unter Rühren zunächst 20,95 g Kupfer(I)jodid, dann tropfenweise eine Lösung von 15,2 g Cyclohept-2-enon (80 %) in 175 ml wasserfreiem Diethylether gegeben. Nach beendeter Zugabe wurde 45 Minuten zum Rückfluß erhitzt. Dann zersetzte man durch Zutropfen von 85 ml einer gesättigten Ammoniumchloridlösung. Nach Verdünnen mit 200 ml Wasser wurde die organische Phase abgetrennt, die wäßrige noch zweimal mit je 100 ml Diethylether extrahiert. Die vereinigten organischen Phasen wurden je einmal mit gesättigten Lösungen von Natriumhydrogencarbonat bzw. Natriumchlorid gewaschen, über Natriumsulfat getrocknet und im Vakuum eingedampft. Man reinigte den Rückstand säulenchromatographisch mit Diethylether/n-Hexan = 1/4, zuletzt 1/1, als Elutionsmittel und erhielt so 16,5 g (68,6 % d.Th.) der Titelverbindung als leicht gelbes Öl.

### 2. Stufe

### 2-Dimethylaminomethyl-6-(3-methoxy-phenyl)-cycloheptanon, Hydrochlorid

Eine Lösung von 16,4 g des Produkts aus Stufe 1 in 150 ml Acetonitril wurde mit 7,2 g N,N-Dimethylmethylenimmoniumchlorid und drei Tropfen Acetylchlorid versetzt und das Gemisch 48 Stunden bei 20°C gerührt. Dann wurde mit 100 ml Diethylether verdünnt, das kristalline Produkt isoliert, mit Diethylether gewaschen und im Vakuum bei 40°C getrocknet. Man erhielt so 21,9 g (93,6 % d.Th.) der Titelverbindung in Form weißer Kristalle.
Schmelzpunkt: 130 - 134,5°C

### 3. Stufe

### 2-Dimethylaminomethyl-1,6-bis-(3-methoxy-phenyl)-cycloheptanol

Zu einer frisch bereiteten Grignardlösung aus 4,42 ml 1-Brom-3-methoxy-benzol und 0,90 g Magnesiumspänen in 35 ml wasserfreiem Tetrahydrofuran wurde bei 20°C unter Rühren eine Lösung von 9,1 g der freien Base des Produkts aus Stufe 2 in 52 ml wasserfreiem Tetrahydrofuran getropft; dann wurde zum Rückfluß erhitzt. Nach beendeter Umsetzung wurde wie in Stufe 1 beschrieben aufgearbeitet. Man erhielt nach säulenchromatographischer Reinigung mit Essigsäureethyl-ester/Methanol = 5/1 als Elutionsmittel 10,44 g (82,4 % d. Th.) der Titelverbindung als nahezu farbloses Öl.

### 4. Stufe

### 3-[2-Dimethylaminomethyl-6-(3-hydroxy-phenyl)-cyclohept-1-enyl]-phenol, Hydrochlorid

10,35 g des Produkts aus Stufe 3 wurden unter Rühren mit 120 ml einer Lösung von Bromwasserstoff in Eisessig (33 % HBr) 5 Stunden auf 100 - 110°C erhitzt. Dann wurde im Vakuum eingedampft, der Rückstand in 150 ml Wasser aufgenommen und mit verdünnter Natronlauge (ca. 5 %) alkalisiert (pH 9 - 10). Man extrahierte dreimal mit je 100 ml Essigsäureethylester, wusch die vereinigten Extrakte einmal mit gesättigter Natrinmchloridlösung, trocknete über Natriumsulfat und dampfte im Vakuum ein. Der Rückstand wurde säulenchromatographisch mit Essigsäureethylester als Elutionsmittel gereinigt.

Man erhielt so 3,71 g (40,7 % % d. Th.) der freien Base der Titelverbindung, die mit Trimethylchlorsilan/Wasser in 2-Butanon ins Hydrochlorid übergeführt wurden.
Schmelzpunkt: ab 110°C unter Zersetzung

### Beispiel 2

Unter Verwendung entsprechender Ausgangsverbindungen ließen sich nach der in Beispiel 1, Stufen 1 - 4, beschriebenen Verfahrensweise, ggf. unter Variation der Reaktionsbedingungen (Lösungsmittel, Temperatur), analog erhalten:
- 2a:: 3-[6-(4-Chlor-phenyl)-2-dimethylaminomethylcyclohept-1-enyl]-phenol, Hydrochlorid
Schmelzpunkt: ab 134°C unter Zersetzung
- 2b:: 3- (2-Dimethylaminomethyl-6-phenyl-cyclohept-1-enyl)-phenol, Hydrochlorid
Schmelzpunkt: 162 - 166°C
- 2c:: 3-(2-Dimethylaminomethyl-6-naphth-1-yl-cyclohept-1-enyl)-phenol, Hydrochlorid
- 2d:: 3-(2-Dimethylaminomethyl-6-naphth-2-yl-cyclohept-1-enyl)-phenol, Hydrochlorid
Schmelzpunkt: 183°C
- 2e:: 3-[2-Dimethylaminomethyl-6-(4-hydroxy-phenyl)-cyclohept-1-enyl]-phenol, Hydrochlorid
Schmelzpunkt: 240 - 242°C
- 2f:: 3-(2-Dimethylaminomethyl-6-m-tolyl-cyclohept-1-enyl)-phenol, Hydrochlorid
Schmelzpunkt: 231 - 233°C
- 2g:: 3-[6-(3-tert-Butyl-phenyl) -2-dimethylaminomethyl-cyclohept-1-enyl]-phenol, Hydrochlorid
Schmelzpunkt: 215 - 218°C
- 2h:: 6-[4-Dimethylaminomethyl-3-(3-hydroxy-phenyl)-cyclohept-3-enyl]-naphth-2-ol, Hydrochlorid
Schmelzpunkt: ab 190°C unter Zersetzung
- 2i:: 3-[2-Dimethylaminomethyl-6-(3-fluor-4-hydroxyphenyl)-cyclohept-1-enyl]-phenol, Hydrochlorid
Schmelzpunkt: 227 - 230°C
- 2j:: 3-[2-Dimethylaminomethyl-6-(2-hydroxy-phenyl)-cyclohept-1-enyl]-phenol, Hydrochlorid
Schmelzpunkt: ab 125°C unter Zersetzung
- 2k:: 3-(6-Cyclohexyl-2-dimethylaminomethyl-cyclohept-1-enyl)-phenol, Hydrochlorid
Schmelzpunkt: 224 - 225,5°C
- 2l:: 3-(6-Cyclohexylmethyl-2-dimethylaminomethylcyclohept-1-enyl)-phenol, Hydrochlorid
Schmelzpunkt: 203 - 206°C
- 2m:: 3-(6-Benzyl-2-dimethylaminomethyl-cyclohept-1-enyl)-phenol, Hydrochlorid
Schmelzpunkt: 208 - 212°C
- 2n:: 3- [2-Dimethylaminomethyl-6-(3-hydroxy-benzyl)-cyclohept-1-enyl)-phenol, Hydrochlorid
Schmelzpunkt: 88°C
- 2o:: 3-(2-Dimethylaminomethyl-6-phenethyl-cyclohept-1-enyl)-phenol, Hydrochlorid
Schmelzpunkt: 188 - 190°C
- 2p:: 3- [2-Dimethylaminomethyl-6-(3,5-dimethyl-4-hydroxy-phenyl)-cyclohept-1-enyl]-phenol, Hydrochlorid
Schmelzpunkt: ab 156°C unter Zersetzung

### Beispiel 3

### 3-{2-[(Methyl-phenethyl-amino)-methyl]-6-phenyl-cyclohept-1-enyl}-phenol, Hydrochlorid

### 1. Stufe

### 2-[(Methyl-phenethyl-amino)-methyl)-6-phenyl-cycloheptanon, Hydrochlorid

Eine Mischung aus 2,77 g 3-Phenyl-cycloheptanon, 2,52 g Methylphenethyl-amin, Hydrochlorid und 1,23 ml einer wäßrigen Formaldehydlösung (36 %) wurde unter starkem Rühren und Überleiten von Stickstoff 2 Stunden auf dem Wasserbad erhitzt. Dann wurde im Vakuum eingedampft, der Rückstand dreimal mit Diethylether/n-Hexan = 1/1 extrahiert und im Vakuum getrocknet. Dabei blieben 5,4 g der rohen Titelverbindung zurück.

### 2. Stufe

### 1-(3-Methoxy-phenyl)-2-[(methyl-phenethyl-amino)-methyl]-6-phenyl-cycloheptanol

4,7 g der freien Base des Produkts aus Stufe 1, 2,76 g 1-Brom-3-methoxy-benzol und 0,4 g Magnesiumspäne wurden wie in Beispiel 1, Stufe 3, beschrieben zur Reaktion gebracht. Nach analoger Aufarbeitung und säulenchromatographischer Reinigung mit Essigsäureethylester/n-Hexan = 1/1 als Elutionsmittel erhielt man 3,1 g (49,9 % % d.Th.) der Titelverbindung als gelbes Öl.

### 3. Stufe

### 3-(2-[(Methyl-phenethyl-amino)-methyl]-6-phenyl-cyclohept-1-enyl}-phenol, Hydrochlorid

2,67 g des Produkts aus Stufe 2 wurde wie in Beispiel 1, Stufe 4, beschrieben mit 27 ml einer Lösung von Bromwasserstoff in Eisessig (33 % HBr) zur Reaktion gebracht. Bei analoger Aufarbeitung fielen 1,24 g (50,2 % d. Th.) der freien Base der Titelverbindung an, die mit Trimethylchlorsilan in 2-Butanon ins Hydrochlorid übergeführt wurden.
Schmelzpunkt: ab 105°C unter Zersetzung

### Beispiel 4

### [2-(3-Methoxy-phenyl)-4-naphth-1-yl-cyclohept-1-enylmethyl]-dimethylamin, Hydrochlorid

4,04 g 2-Dimethylaminomethyl-1-(3-methoxy-phenyl)-6-naphth-1-yl-cycloheptanol (Produkt aus Beispiel 2c, Stufe 3) wurden 24 Stunden bei 50° C mit 50 ml 6 N Salzsäure gerührt. Man alkalisierte mit Natronlauge und extrahierte dreimal mit je 50 ml Essigsäureethylester. Die Extrakte wurden mit gesättigter Natriumchloridlösung gewaschen, über Natriumsulfat getrocknet und im Vakuum eingedampft. Den Rückstand reinigte man säulenchromatographisch mit Essiqsäureethylester/Methanol = 4/1 als Elutionsmittel, wobei 2,94 g (76,3 d.Th.) der freien Base der Titelverbindung anfielen, die mit Trimethylchlorsilan/Wasser in 2-Butanon ins Hydrochlorid übergeführt wurden.

## Patentansprüche

1. Substituierte Cycloheptene der allgemeinen Formel I, 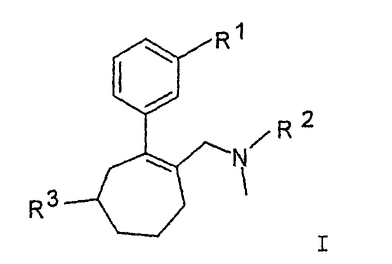 worin
R¹ OH, O-(C₁₋₆)-Alkyl, O-(C₃₋₇)Cycloalkyl, O-Aryl, C₁₋₆-Alkyl-COO-, Aryl-COO-,
R² C₁₋₆-Alkyl, (CH₂)₍₁₋₂₎-Aryl, C₂₋₆-Alkenylen-Aryl und
R³ (CH₂)₍₀₋₁₎-C₅₋₇-Cycloalkyl, (CH₂)₍₀₋₂₎-Aryl, Heterocyclyl, C₁₋₆-Alkyl-Heterocyclyl
bedeuten oder in Form ihrer Enantiomeren, Diastereomeren, Racemate, Basen oder als Salze von physiologisch verträglichen Säuren, und
wobei der Ausdruck "Aryl" Naphthyl oder unsubstituierte oder ein- oder mehrfach mit OH, F, Cl, CF₃, C₁₋₆-Alkyl, C₁₋₆-Alkoxy, C₁₋₇-Cycloalkoxy, C₃₋₇-Cycloalkyl, C₂₋₆-Alkenylen Heterocyclyl oder Phenyl substituierte Phenyle bedeutet und die Heterocyclyl- oder Phenylreste gegebenenfalls ankondensiert sein, können
und der Ausdruck "Heterocyclyl" 5- oder 6-gliedrige gesättigte oder ungesättigte, gegebenenfalls mit einem ankondensierten Arylsystem versehene, heterocyclische Verbindungen, die 1 oder 2 Heteroatome aus der Gruppe von Stickstoff, Sauersroff und/oder Schwefel enthalten, bedeutet.

2. Substituierte Cycloheptene gemäß Anspruch 1, **dadurch gekennzeichnet, daß** R¹ OH, O-(C₁₋₆)-Alkyl, O-(C₃₋₇)-Cycloalkyl, O-Aryl, C₁₋₆-Alkyl-COO- oder Aryl-COOund R² bis R³ die Bedeutung gemäß der Definition der allgemeinen Formel I haben.

3. Substituierte Cycloheptene gemäß den Ansprüchen 1 oder 2, **dadurch gekennzeichnet, daß** R¹ OH, O-(C₁₋₆)-Alkyl oder O-(C₃₋₇)-Cycloalkyl, R² C₁₋₆-Alkyl oder (CH₂) ₍₁₋₂₎-Aryl und R³ die Bedeutung gemäß der Definition der allgemeinen Formel I haben.

4. Substituierte Cycloheptene gemäß den Ansprüchen 1 bis 3, **dadurch gekennzeichnet, daß** R¹ OH, R² C₁₋₆-Alkyl oder (CH₂)₍₁₋₂₎-Aryl und R³ die Bedeutung gemäß der Definition der allgemeinen Formel I haben.

5. Substituierte Cycloheptene gemäß den Ansprüchen 1 bis 4, **dadurch gekennzeichnet, daß** R¹ OH, R² C₁₋₆-Alkyl und R³ die Bedeutung gemäß der Definition der allgemeinen Formel I haben.

6. Verbindungen gemäß Anspruch 1 ausgewählt aus der Gruppe:
3-[6-(4-Chlor-phenyl)-2-dimethylaminomethyl-cyclo-hept-1-enyl]-phenol, Hydrochlorid,
3-(2-Dimethylaminomethyl-6-phenyl-cyclohept-1-enyl)-phenol, Hydrochlorid,
3-(2-Dimethylaminomethyl-6-naphth-1-yl-cyclohept-1-enyl)-phenol, Hydrochlorid,
3-(2-Dimethylaminomethyl-6-naphth-2-yl-cyclohept-1-enyl)-phenol, Hydrochlorid,
3-[2-Dimethylaminomethyl-6-(4-hydroxy-phenyl)-cyclohept-1-enyl]-phenol, Hydrochlorid,
3-(2-Dimethylaminomethyl-6-m-tolyl-cyclohept-1-enyl)-phenol, Hydrochlorid,
3-[6-(3-tert-Butyl-phenyl)-2-dimethylaminomethyl-cyclohept-1-enyl]-phenol, Hydrochlorid,
6-[4-Dimethylaminomethyl-3-(3-hydroxy-phenyl)-cyclohept-3-enyl]-naphth-2-ol, Hydrochlorid,
3-[2-Dimethylaminomethyl-6-(3-fluor-4-hydroxy-phe-nyl)-cyclohept-1-enyl]-phenol, Hydrochlorid,
3-[2-Dimethylaminomethyl-6-(2-hydroxy-phenyl)-cyclohept-1-enyl]-phenol, Hydrochlorid,
3-(6-Cyclohexyl-2-dimethylaminomethyl-cyclohept-1-enyl)-phenol, Hydrochlorid,
3-(6-Cyclohexylmethyl-2-dimethylaminomethyl-cyclo-hept-1-enyl)-phenol, Hydrochlorid,
3-(6-Benzyl-2-dimethylaminomethyl-cyclohept-1-enyl)-phenol, Hydrochlorid,
3-[2-Dimethylaminomethyl-6-(3-hydroxy-benzyl)-cyclohept-1-enyl)-phenol, Hydrochlorid,
3-(2-Dimethylaminomethyl-6-phenethyl-cyclohept-1-enyl)-phenol, Hydrochlorid,
3-[2-Dimethylaminomethyl-6-(3,5-dimethyl-4-hydroxy-phenyl)-cycLohept-1-enyl]-phenol, Hydrochlorid,
3-[2-Dimethylaminomethyl-6-(3-hydroxy-phenyl)-cyclohept-1-enyl]-phenol, Hydrochlorid,
3-{2-[(Methyl-phenethyl-amino)-methyl]-6-phenyl-cyclohept-1-enyl}-phenol, Hydrochlorid und
[2-(3-Methoxy-phenyl)-4-naphth-1-yl-cyclohept-1-enylmethyl]-dimethylamin, Hydrochlorid.

7. Verfahren zur Herstellung einer Verbindung der Formel(I) 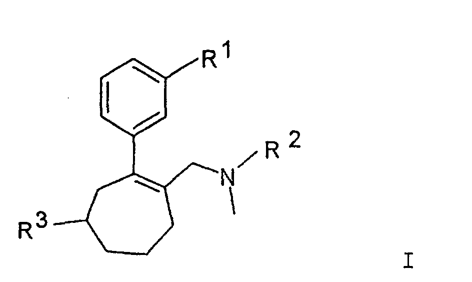 worin
R¹ bis R³ die Bedeutung gemäß Anspruch 1 haben, **dadurch gekennzeichnet, daß** ein tertiärer Alkohol der allgemeinen Formel (II) 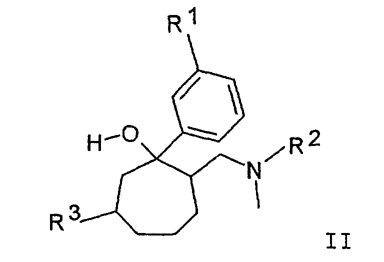 worin R¹ bis R³ dieselbe Bedeutung wie in Formel (I) haben, mit Säuren in einem Temperaturbereich von 20° C bis 110° C umgesetzt werden, wobei tertiäre Alkohole der allgemeinen Formel (II) dadurch erhalten werden, daß zunächst Aminoketone der allgemeinen Formel (III) 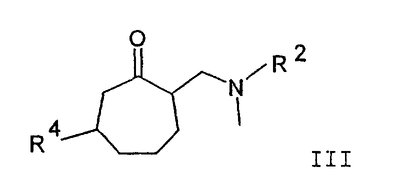 worin R² die Bedeutung gemäß der allgemeinen Formel I besitzt und R⁴ wie R³ definioert ist, mit der Ausnahme, daß eine vorhandene Hydroxy-Funktion in geschützter Form als Benzyloxy- oder Silanyloxygruppe vorliegt, mit einer metallorganischen Verbindung der Formel IV, 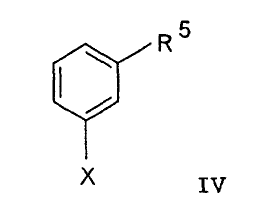 worin X für MgCl, MgBr, MgI oder Li steht und R⁵ die Bedeutung gemäß R¹ hat, mit der Ausnahme, daß eine vorhandene Hydroxy-Funktion in geschützter Formm als Benzyloxy- oder Silanyloxygruppe vorliegt, die zu einer Verbindung der Formel (IIa) 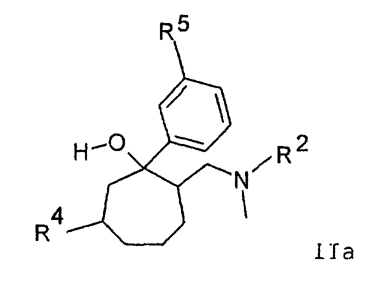 umgesetzt wird und diese dann in eine Verbindung der allgemeinen Formel (II) überführt wird.

8. Arzneimittel enthaltend ein substituiertes Cyclohepten der allgemeinen Formel (I) nach Anspruch 1 als Wirkstoff.

9. Verwendung eines substituierten Cycloheptens der allgemeinen Formel (I) nach Anspruch 1 zur Herstellung eines Analgetikums.

## Claims

1. Substituted cycloheptenes of the general formula I 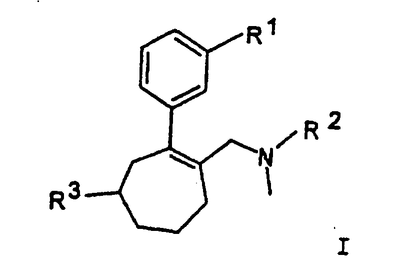 wherein
R¹ denotes OH, O-(C₁₋₆)-alkyl, O- (C₃₋₇) cycloalkyl, O-aryl, C₁₋₆-alkyl-COO-, aryl-COO-,
R² denotes C₁₋₆-alkyl, (OH₂)₍₁₋₂₎-aryl, C₂₋₆-alkenylene-aryl and
R³ denotes (CH₂)₍₀₋₁₎-C₅₋₇-cycloalkyl, (CH₂)₍₀₋₂₎-aryl, heterocyclyl, C₁₋₆-alkyl-heterocyclyl
or their enantiomers, diastereomers, racemates, bases or salts of physiologically acceptable acids, and
wherein the term "aryl" denotes naphthyl, or phenyls which are unsubstituted or mono- or polysubstituted by OH, F, Cl, CF₃, C₁₋₆-alkyl, C₁₋₆-alkoxy, C₁₋₇-cycloalkoxy, C₃₋₇-cycloalkyl, C₂₋₆-alkenylene, heterocyclyl or phenyl and the heterocyclyl or phenyl radicals can optionally be fused-on,
and the term "heterocyclyl" denotes 5- or 6-membered saturated or unsaturated heterocyclic compounds which are optionally provided with a fused-on aryl system and contain 1 or 2 heteroatoms from the group consisting of nitrogen, oxygen and/or sulfur.

2. Substituted cycloheptenes according to claim 1, **characterized in that** R¹ denotes OH, O-(C₁₋₆)-alkyl, O-(C₃₋₇)-cycloalkyl, O-aryl, C₁₋₆-alkyl-COO- or aryl-COO-and R² to R³ have the meaning according to the definition of the general formula I.

3. Substituted cycloheptenes according to claims 1 or 2, **characterized in that** R¹ denotes OH, O-(C₁₋₆)-alkyl or O-(C₃₋₇)-cycloalkyl, R² denotes C₁₋₆-alkyl or (CH₂)₍₁₋₂₎-aryl and R³ has the meaning according to the definition of the general formula I.

4. Substituted cycloheptenes according to claims 1 to 3, **characterized in that** R¹ denotes OH, R² denotes C₁₋₆-alkyl or (CH₂)₍₁₋₂₎-aryl and R³ has the meaning according to the definition of the general formula I.

5. Substituted cycloheptenes according to claims 1 to 4, **characterized in that** R¹ denotes OH, R² denotes C₁₋₆-alkyl and R³ has the meaning according to the definition of the general formula I.

6. Compounds according to claim 1, selected from the group consisting of:
3-[6-(4-chloro-phenyl)-2-dimethylaminomethyl-cyclohept-1-enyl]-phenol, hydrochloride,
3-(2-dimethylaminomethyl-6-phenyl-cyclohept-1-enyl)-phenol, hydrochloride,
3-(2-dimethylaminomethyl-6-naphth-1-yl-cyclohept-1-enyl)-phenol, hydrochloride,
3-(2-dimethylaminomethyl-6-naphth-2-yl-cyclohept-1-enyl)-phenol, hydrochloride,
3-[2-dimethylaminomethyl-6-(4-hydroxy-phenyl)-cyclohept-1-enyl]-phenol, hydrochloride,
3-(2-dimethylaminomethyl-6-m-tolyl-cyclohept-1-enyl)-phenol, hydrochloride,
3-[6-(3-tert-butyl-phenyl)-2-dimethylaminomethyl-cyclohept-1-enyl]-phenol, hydrochloride,
6-[4-dimethylaminomethyl-3-(3-hydroxy-phenyl)-cyclohept-3-enyl]-naphth-2-ol, hydrochloride,
3-[2-dimethylaminomethyl-6-(3-fluoro-4-hydroxy-phe-nyl)-cyclohept-1-enyl]-phenol, hydrochloride,
3-[2-dimethylaminomethyl-6-(2-hydroxy-phenyl)-cyclohept-1-enyl]-phenol, hydrochloride,
3-(5-cyclohexyl-2-dimethylaminomethyl-cyclohept-1-enyl)-phenol, hydrochloride,
3-(6-cyclohexylmethyl-2-dimethylaminomethyl-cyclo-hept-1-enyl) -phenol, hydrochloride,
3-(6-benzyl-2-dimethylaminomethyl-cyclohept-1-enyl)-phenol, hydrochloride,
3-[2-dimethylaminomethyl-6-(3-hydroxy-benzyl)-cyclohept-1-enyl)-phenol, hydrochloride,
3-(2-dimethylaminomethyl-6-phenethyl-cyclohept-1-enyl)-phenol, hydrochloride,
3-[2-dimethylaminomethyl-6-(3,5-dimethyl-4-hydroxy-phenyl)-cyclohept-1-enyl)-phenol, hydrochloride,
3-[2-dimethylaminomethyl-6-(3-hydroxy-phenyl)-cyclohept-1-enyl]-phenol, hydrochloride,
3-{2-[(methyl-phenethyl-amino)-methyl)-6-phenyl-cyclohept-1-enyl}-phenol, hydrochloride and
[2-(3-methoxy-phenyl)-4-naphth-1-yl-cyclohept-1-enyl-methyl] -dimethylamine, hydrochloride.

7. Process for the preparation of a compound of the formula (I) 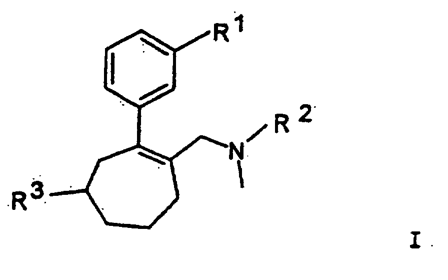 wherein
R¹ to R³ have the meaning according to claim 1, **characterized in that** a tertiary alcohol of the general formula (II) 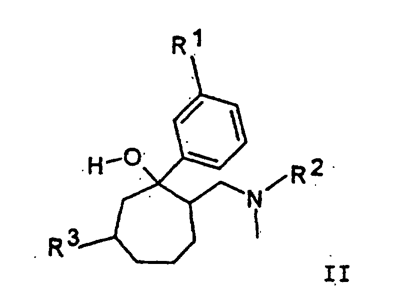 wherein R¹ to R³ have the same meaning as in formula (I), is reacted with acids in a temperature range from 20°C to 110°C, tertiary alcohols of the general formula (II) being obtained by a process in which amino-ketones of the general formula (III) 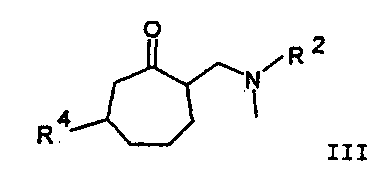 wherein R² has the meaning according to the general formula I and R⁴ is defined as R³, with the exception that a hydroxyl function present is present in protected form as the benzyloxy or silanyloxy group, are first reacted with an organometallic compound of the formula IV 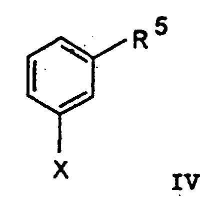 wherein X represents MgCl, MgBr, MgI or Li and R⁵ has the meaning according to R¹, with the exception that a hydroxyl function present is present in protected form as the benzyloxy or silanyloxy group, which to give a compound of the formula (IIa) 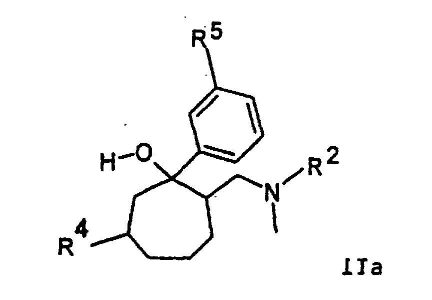 and this is then converted into a compound of the general formula (II).

8. Medicaments comprising a substituted cycloheptene of the general formula (I) according to claim 1 as the active compound.

9. Use of a substituted cycloheptene of the general formula (I) according to claim 1 for the preparation of an analgesic.

## Revendications

1. Cycloheptènes substitués de formule générale I, 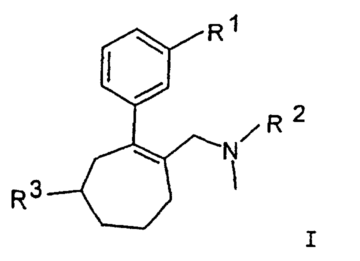 dans laquelle
R¹ est un groupe OH, O-(alkyle en C₁ à C₆), O-(cycloalkyle en C₃ à C₇), O-aryle, (alkyle en C₁ à C₆) -COO-, aryl-COO-,
R² est un groupe alkyle en C₁ à C₆, (CH₂)₍₁₋₂₎-aryle, (alcénylène en C₂ à C₆)-aryle et
R³ est un groupe (CH₂)₍₀₋₁₎-(cycloalkyle en C₅ à C₇), (CH₂)₍₀₋₂₎-aryle, hétérocyclyle, (alkyle en C₁ à C₆)-hétérocyclyle,
ou sous la forme de leurs énantiomères, de leurs diastéréo-isomères, de leurs racémates, de leurs bases ou comme sels d'acides acceptables du point de vue physiologique, le terme "aryle" désignant un groupe naphtyle ou des groupes phényle non substitués ou portant un ou plusieurs substituants OH, F, Cl, CF₃, alkyle en C₁ à C₆, alkoxy en C₁ à C₆, cycloalkoxy en C₁ à C₇, cycloalkyle en C₃ à C₇, alcénylène en C₂ à C₆, hétérocyclyle ou phényle et les restes hétérocyclyle ou phényle pouvant éventuellement être condensés
et le terme "hétérocyclyle" désignant des composés hétérocycliques pentagonaux ou hexagonaux saturés ou non saturés, éventuellement pourvus d'un noyau aryle condensé et ayant un ou deux hétéro-atomes du groupe azote, oxygène et/ou soufre.

2. Cycloheptènes substitués suivant la revendication 1, **caractérisés en ce que** R¹ est un groupe OH, O-(alkyle en C₁ à C₆), O-(cycloalkyle en C₃ à C₇), O-aryle, (alkyle en C₁ à C₆)-COO- ou aryl-COO-, et R² et R³ ont une signification correspondant à la définition de la formule générale I.

3. Cycloheptènes substitués suivant les revendications 1 ou 2, **caractérisés en ce que** R¹ est un groupe OH, O-(alkyle en C₁ à C₆) ou O-(cycloalkyle en C₃ à C₇), R² est un groupe alkyle en C₁ à C₆ ou (CH₂)₍₁₋₂₎-aryle, et R³ a la signification correspondant à la définition de la formule générale I.

4. Cycloheptènes substitués suivant les revendications 1 à 3, **caractérisés en ce que** R¹ est un groupe OH, R² est un groupe alkyle en C₁ à C₆ ou (CH₂)₍₁₋₂₎-aryle et R³ a une signification correspondant à la définition de la formule générale I.

5. Cycloheptènes substitués suivant les revendications 1 à 4, **caractérisés en ce que** R¹ est un groupe OH, R² est un groupe alkyle en C₁ à C₆ et R³ a une signification correspondant à la définition de la formule générale I.

6. Composés suivant la revendication 1, choisis dans le groupe des composés suivants :
chlorhydrate de 3-[6-(4-chlorophényl)-2-diméthylaminométhyl-cyclohept-1-ényl]phénol,
chlorhydrate de 3-(2-diméthylaminométhyl-6-phényl-cyclohept-1-ényl)phénol
chlorhydrate de 3-(2-diméthylaminométhyl-6-napht-1-yl-cyclohept-1-ényl)phénol,
chlorhydrate de 3-(2-diméthylaminométhyl-6-napht-2-yl-cyclohept-1-ényl)phénol,
chlorhydrate de 3-[2-diméthylaminométhyl-6-(4-hydroxyphényl)cyclohept-1-ényl]phénol,
chlorhydrate de 3-(2-diméthylaminométhyl-6-m-tolyl-cyclohept-1-ényl)phénol,
chlorhydrate de 3-[6-(3-tertiobutylphényl)-2-diméthylaminométhyl-cyclohept-1-ényl]phénol,
chlorhydrate de 6-[4-diméthylaminométhyl-3-(3-hydroxyphényl)cyclohept-3-ényl]-napht-2-ol,
chlorhydrate de 3-[2-diméthylaminométhyl-6-(3-fluoro-4-hydroxyphényl)cyclohept-1-ényl]phénol,
chlorhydrate de 3-[2-diméthylaminométhyl-6-(2-hydroxyphényl)cyclohept-1-ényl]phénol,
chlorhydrate de 3-(6-cyclohexyl-2-diméthylaminométhyl-cyclohept-1-ényl)phénol,
chlorhydrate de 3- (6-cyclohexylméthyl-2-diméthylaminométhyl-cyclohept-1-ényl)phénol,
chlorhydrate de 3-(6-benzyl-2-diméthylaminométhyl-cyclohept-1-ényl)phénol,
chlorhydrate de 3-[2-diméthylaminométhyl-6-(3-hydroxybenzyl)cyclohept-1-ényl)phénol,
chlorhydrate de 3-(2-diméthylaminométhyl-6-phénéthyl-cyclohept-1-ényl)phénol,
chlorhydrate de 3- [2-diméthylaminométhyl-6- (3,5-diméthyl-4-hydroxyphényl)cyclohept-1-ényl]phénol,
chlorhydrate de 3-[2-diméthylaminométhyl-6-(3-hydroxyphényl)cyclohept-1-ényl]phénol,
chlorhydrate de 3-{2-[(méthylphénéthylamino)-méthyl]-6-phényl-cyclohept-1-ényl}phénol, et
chlorhydrate de [2-(3-méthoxyphényl)-4-napht-1-yl-cyclohept-1-ényl-méthyl]diméthylamine.

7. Procédé de production d'un composé de formule (I) 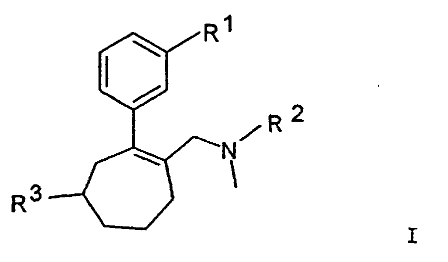 dans laquelle
R¹ à R³ ont la définition suivant la revendication 1, **caractérisé en ce qu'**on fait réagir un alcool tertiaire de formule générale (II) 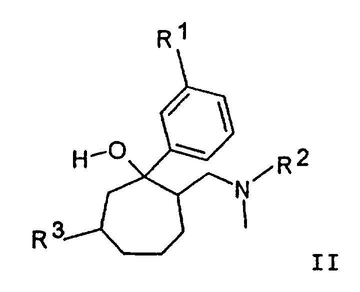 dans laquelle R¹ à R³ ont la même définition que dans la formule (I), avec des acides dans une plage de températures de 20°C à 110°C, les alcools tertiaires de formule générale (II) étant obtenus en faisant réagir tout d'abord des aminocétones de formule générale (III) 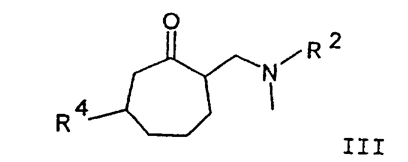 dans laquelle R² possède la définition suivant la formule générale I et R⁴ est défini comme R³, excepté qu'une fonction hydroxy existe sous la forme protégée comme groupe benzyloxy ou silanyloxy, avec un composé organométallique de formule IV, 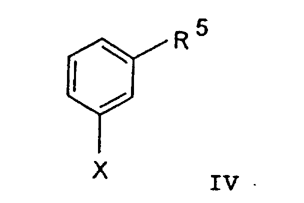 dans laquelle X représente MgCl, MgBr, MgI ou Li et R⁵ a la définition correspondant à R¹, excepté qu'une fonction hydroxy présente existe sous la forme protégée comme groupe benzyloxy ou silanyloxy, pour obtenir un composé de formule (IIa) 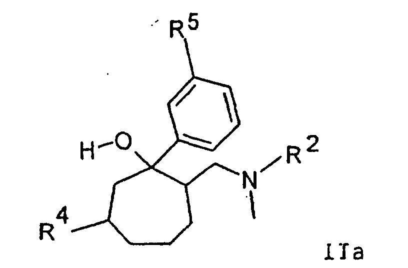 qu'on transforme ensuite en un composé de formule générale (II).

8. Médicament contenant comme substance active un cycloheptène substitué de formule générale (I) suivant la revendication 1.

9. Utilisation d'un cycloheptène substitué de formule générale I suivant la revendication 1, pour la préparation d'un analgésique.
